# EUROPEAN PATENT APPLICATION

(11) **EP 0 824 919 A2**
(43) Date of publication of application: **25.02.1998**
(21) Application number: 97306203.7
(22) Date of filing: 15.08.1997
(51) Int. Cl.: A61K 41/00

(54) **Therapeutic methods and compositions for use therein**

(30) Priority: 19.08.1996 GB 9617346
(71) Applicant: Holt, John Alfred Gorton, Perth, Western Australia 6009 (AU)
(72) Inventor: Holt, John Alfred Gorton, Perth, Western Australia 6009 (AU)
(74) Representative: Brown, David Leslie

(57) **Abstract**

There is described a therapeutic method, applicable *in vivo* to a patient, which comprises administering to the patient, body fluid or body part an effective amount of S-methyl cysteine sulfoximine of the formula 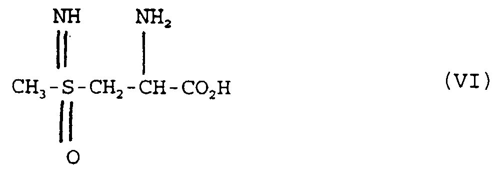 or a non-toxic salt thereof, and while said sulfoximine is present administering to the patient an effective does of microwave electromagnetic radiation of frequency in the range of about 400-450 MHz.

In a modification of the method, a combination comprising methionine sulfoximine (or a non-toxic salt thereof) and methionine (or a non-toxic salt thereof) may be used in place of or in addition to the sulfoximine of formula VI.

The method is effective to treat cancers present in the patient, to enhance T-cell count in an immunodeficient individual, and to reduce concentrations of pathogenic viral and/or prion particles in body tissue and body fluids, and/or other particles which are actively growing exponentially *in vivo.*

## Description

The present invention relates to therapeutic methods and compositions for use therein.

European Patent Application No. 0531031 (the disclosure of which is incorporated herein by reference) describes a cancer therapy, and compositions for use therein, in which an effective amount of a non-toxic organic disulfide is administered to a patient and while said disulfide is present at a cancer site of the patient there is administered to the cancer site an effective dose of microwave electromagnetic radiation of frequency in the range of about 400-450 MHz.

European Patent Application No. 0616803 (the disclosure of which is incorporated herein by reference) describes the use of such methods in enhancing blood T-cell count in immunodeficient (e.g. HIV-positive) individuals. In both documents, cystine, which has the formula 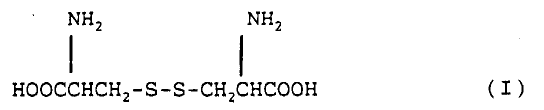 and non-toxic salts thereof are stated to be preferred disulfide agents.

Oxidised glutathione 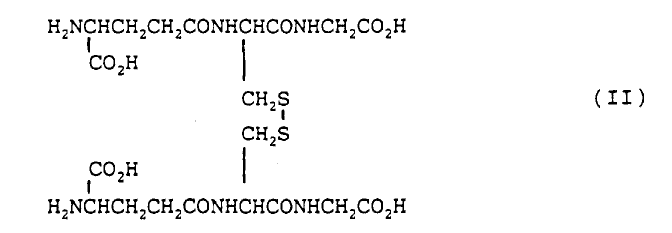 and non-toxic salts thereof are stated in EP-A-0616803 to be alternative preferred disulfide agents in the context of enhancement of a patient's T-cell count.

T-butyl hydroperoxide, which has the formula

CH₃C(CH₃)₂OOH (III)

is stated in EP-A-0531031 to be desirably used as an oxidising agent to prevent degradation of the disulfide agents.

EP-A-0531031 generically describes the use of non-toxic organic sulfoximines, preferably methionine sulfoximine 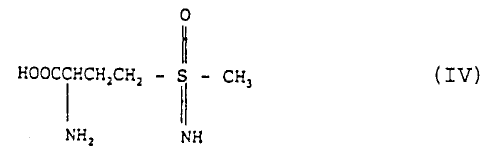 in the above-mentioned cancer therapy, in place of or additional to the organic disulfide active agents, although no specific example of such a use is given.

European Patent Application No. 0705603 (the disclosure of which is incorporated herein by reference) describes modifications of the above methods in which a non-toxic cysteine sulfoxide of formula 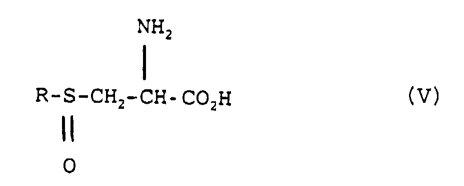 or a non-toxic salt thereof, in which R is a C₁₋₄ alkyl or a C₂₋₄ alkenyl group, is used in place of, or in addition to, the disulfide active agents of the prior art methods mentioned above to provide a therapeutic method (e.g. an anticancer therapy or a method for enhancing T-cell count) applicable *in vivo* to a patient or *in vitro* to a transfusable body fluid or transplantable body part. The active agent(s) is/are administered to the patient, body fluid or body part in an effective amount and, while present, there is administered to the patient, body fluid or body part an effective dose of microwave electromagnetic radiation of frequency in the range of about 400-450 MHz. The compounds in which R is methyl, ethyl, propyl or butyl are stated to be preferred.

T-butyl hydroperoxide (III) is stated in EP-0705603 to be desirably used as an oxidising agent preceding the administration of the active agent(s).

European Patent Application No. 0764442 describes the use of such disulfides and sulfoxides in reducing the concentration of intracellular pathogenic viral and/or prion particles in body tissue and fluids.

The present invention is based on my surprising finding that the above methods may be performed at least as well by using S-methyl cysteine sulfoximines in place of, or in addition to, the previously described active agents.

The invention may therefore be stated to provide in one aspect a therapeutic method which comprises administering to a patient an effective amount of a non-toxic agent selected from S-methyl cysteine sulfoximine of the formula 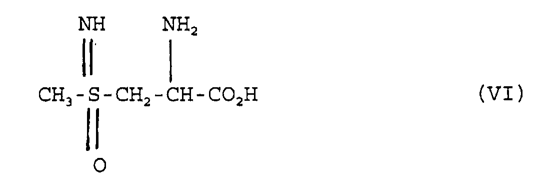 and non-toxic salts thereof, and while said non-toxic agent is present administering to the patient an effective dose of microwave electromagnetic radiation of frequency in the range of about 400-450 MHz.

More specifically, the invention provides a method of cancer therapy in a patient which comprises administering to a cancer site of the patient an effective amount of a S-methyl cysteine sulfoximine or a non-toxic salt thereof, and while said sulfoximine is present at the cancer site of the patient administering to the cancer site an effective dose of microwave electromagnetic radiation of frequency in the range of about 400-450 MHz.

According to a further specific form, the invention provides a method for enhancing T-cell count in an immunodeficient individual, which comprises administering to the individual an effective amount of S-methyl cysteine sulfoximine or a non-toxic salt thereof, and while said sulfoximine is present in the individual administering to the individual an effective dose of microwave electromagnetic radiation of frequency in the range of about 400-450 MHz.

According to a further specific form of the invention, there is provided an *in vivo* method for reducing the concentration of pathogenic viral and/or prion particles in body tissue and body fluids, in which S-methyl cysteine sulfoximine or a non-toxic salt thereof is administered to the body tissue or body fluid and the microwave radiation subsequently administered to the body tissue or body fluid.

According to a further aspect, the invention provides the use of S-methyl cysteine sulfoximine or a non-toxic salt thereof in the preparation of a composition or compositions for use in a therapeutic method according to the invention as described above.

The expression "non-toxic" used herein refers to an acceptably low level of toxicity when a compound is present at an effective amount, and not necessarily to a complete absence of toxic effects. In particular, compounds which have only a temporary toxic effect and do no significant permanent harm, or for which any toxic effects can readily be counteracted by the administration of a non-toxic "antidote" agent, will be referred to and understood as "non-toxic" herein.

The expression "cancer" used herein refers to all forms of malignant neoplasia. There may particularly be mentioned a malignant neoplasm of the epithelium (for example, a carcinoma such as squamous cell carcinoma, basal cell carcinoma, transitional cell carcinoma, adenocarcinoma or cystadenocarcinoma), pigment cells (for example, a malignant melanoma), neural and brain tissue (for example, neurofibrosarcoma, malignant glioma, glioblastoma, medulloblastoma or neuroblastoma), kidney tissue (for example, a nephroblastoma), eye tissue (for example, a retinoblastoma), connective tissue (for example, a sarcoma or meningioma such as fibrosarcoma, liposarcoma, Kaposi's sarcoma, chondrosarcoma, osteosarcoma, myosarcoma, leiomyosarcoma, rhabdomyosarcoma, angiosarcoma, haemangiosarcoma, lymphangiosarcoma or malignant meningioma), lymphoreticular and haematopoietic tissue (for example, a malignant non-Hodgkin's lymphoma, lymphocytic leukaemia, plasmacytoma and multiple myeloma, reticulum cell sarcoma, Hodgkin's disease, granulocytic leukaemia or monocytic leukaemia) or embryonic-originating tissue (for example, a malignant teratoma, teratocarcinoma or chorioncarcinoma).

The expression "cancer site" is not intended to be restricted to localised sites within a patient's body, but covers widespread and multiple sites such as occur, for example, in leukaemias or in advanced cancerous invasions.

The expression "immunodeficient" used herein refers to all forms of reduced or impaired immune function of viral origin, for example human immunodeficiency virus (HIV) infection or acquired immunodeficiency syndrome (AIDS).

The expression "pathogenic viral and/or prion particles" used herein includes all disease-causing infective particulate agents having a size smaller than bacterial pathogens, which are growing exponentially in vivo. Prion particles are generally smaller than viral particles.

Virus- and prion-propagated diseases treatable by the method of the invention include acquired immunodeficiency syndrome (AIDS) , scleroderma, ankylosing spondylitis, bovine spongiform encephalopathy (BSE or "Mad Cow" disease), scrapie, hepatitis, disseminated ("multiple") sclerosis (MS) and Creutzfeld Jacob disease (CJD). The corresponding viral and/or prion particles are destroyed when growing exponentially, by the method of the invention.

Thus, in preferred aspects, the invention provides a therapeutic method for reducing the concentration of infective particulate agents selected from human immunodeficiency virus (HIV), scleroderma agent, ankylosing spondylitis agent, BSE agent, scrapie agent, hepatitis agent, MS agent and CJD agent in body tissue and fluids, and the use of the active agent(s) as defined above in the preparation of a composition or compositions for use in such a method.

If desired, more than one active agent as defined above may be used simultaneously or sequentially according to the invention. There may also be used additional active agents selected from active agents described in EP-A-0531031 and EP-A-0616803, non-toxic cysteine sulfoxides of formula V and non-toxic salts thereof. Mixtures of such compounds may also be used. It is not necessary, in the method of the present invention, to use oxidising agents to protect the sulfoximine active agent (s) against degradation.

An optically active form of the S-methyl cysteine sulfoximine, or alternatively a racemic mixture, may be used. The laevorotatory (L) form is preferred. The non-toxic salts are preferably non-toxic acid or base addition salts, for example addition salts of strong acids or bases.

S-methyl-L-cysteine sulfoximine, S-methyl-L-cysteine sulfoxide, S-ethyl-L-cysteine sulfoxide, S-propyl-L-cysteine sulfoxide, S-butyl-L-cysteine sulfoxide and the non-toxic salts of any of the above listed compounds, are non-toxic in doses exceeding 100 mg per kilogram.

The active agent may be administered in the form of methionine sulfoximine (IV) or a non-toxic salt thereof. The optical form DL-methionine DL-sulfoximine is preferred. Methionine sulfoximine is metabolised in the brain to S-methyl cysteine sulfoximine. However, methionine sulfoximine is too toxic to be an agent of first choice, unless administered in association with methionine: 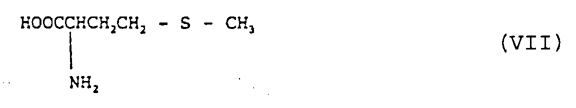 or a non-toxic salt thereof.

According to a further aspect of the present invention, therefore, there is provided a modification of the methods and uses of the present invention as described above, in which, in place of or in addition to the agent(s) described above, there is used a combination comprising methionine sulfoximine (IV) or a non-toxic salt thereof and methionine (VII) or a non-toxic salt thereof.

The composition form(s) by which the active agent(s) is or are administered may take any suitable form and employ any suitable conventional pharmaceutical carrier(s) or excipient(s). The active agent (s) should generally be administered parenterally. In the case of methyl cysteine sulfoximine, oral or intravenous administration may be equally used. The intravenous dose, preferably immediately before therapy, is between about 15-35 mg, preferably about 25 mg per kilogram body weight. The oral dose, preferably about one hour before therapy during fasting, is approximately double the intravenous dose. In the case of methionine sulfoximine, a dose of about 1 mg per Kg body weight per day is preferably used. The daily dose of the methionine is preferably in the range of about 0.5 to 2 grams, preferably administered after the methionine sulfoximine.

The solution or other composition form may if desired include additional components such as salts (e.g. sodium chloride), solubilising and/or suspending agents and the like.

A suitable composition for use in a method of therapy according to the invention thus comprises an aqueous solution of S-methyl cysteine sulfoximine (or a non-toxic salt thereof) at a concentration of up to approximately 120 g/l (e.g. approximately 20-100 g/l).

The composition may suitably take the form of a kit of two or more compositions containing the agents, preferably selected from S-methyl cysteine sulfoximine, methionine sulfoximine/methionine, compounds of formula V, and non-toxic salts thereof, for rapidly sequential administration in a method of therapy according to the invention, a first composition comprising an aqueous solution containing a first agent selected from the agents as defined above, and a second composition comprising an aqueous solution containing a second agent selected from the agents as defined above, with the provisos that at least one of said first and second active agents is S-methyl cysteine sulfoximine, methionine sulfoximine/methionine or a salt thereof; the said first and second compositions being optionally together with further composition(s) and instructional literature for the therapy.

The compositions are prepared by standard mixing techniques that will be readily apparent to those skilled in this art. In the case of sparingly soluble active agents a suitable solubilising agent should first be dissolved in the aqueous medium.

Aqueous solutions of the active agent of formula VI and their salts at concentrations of up to around 100 grams per litre are convenient, enabling a daily injection volume of 50-100 ml to deliver around 1 to 5 grams of the desired sulfoximine orally or intravenously.

If one or more compound of formula V is present in a separate solution, an aqueous solution at a concentration of up to around 100 grams per litre is convenient, enabling a daily injection volume of 50-60 ml to deliver around 5 grams of the desired sulfoxide(s) intravenously.

After administration of the active agent(s) according to the invention and any required oxidising agent(s), the microwave radiation should normally be begun to be administered between about 1 and 5 minutes later.

To administer *in vivo* the microwave radiation, preferably over the entire body of the patient, the patient is brought close to a suitable number of microwave antennae (e.g. 3 or 4 antennae arranged to encircle the patient's body) and the antennae energised to transmit UHF microwave radiation. Normally the patient may conveniently be moved under the antennae. In the case of cancer therapy at least the cancer site(s) will be covered, or optionally the entire body of the patient if that is most convenient. Normally in the case of cancer therapy an area of at least about 30 cm radius around each cancer site should be irradiated, and for this purpose the patient may conveniently be moved under the antennae.

The total antennae power should preferably be less than about 4 kW (e.g. adjustable between about 1 and about 2 kW) in order to remain tolerable to the average patient. Patients may preferably be treated in two or more sessions, preferably three sessions, per day at daily intervals or every other or every third day for a treatment period of up to about four weeks.

Preferably, each session involves administration of the active agent(s) followed over the subsequent 30 minutes by from 1 to about 5 exposures (e.g. three exposures) to the microwave radiation, each exposure to the radiation lasting for about 5 to 10 minutes, with a 2 to 5 minute rest period between exposures. The total length of treatment on each session day will be dependent on the length of time over which the agent(s) remain in the patient's body without substantial loss, and the length of rests between exposures which the patient requires. For optimum results each session should be terminated within 30 minutes of the first agent being administered (by intravenous injection) . Small bodies (e.g. children) and adults can normally tolerate the same radiation power of about 1.6 to 2 kW per session.

As mentioned above, the microwave radiation should be in the frequency range of about 400-450 MHz. More preferably, the frequency should be in the range of about 425-450 MHz, most preferably 432-436 MHz (e.g. about 434 MHz).

The antennae are suitably standard commercially available microwave antennae of a convenient size and shape to accommodate the shape of the patient's body. Each antenna may consist of a single circular turn approximately 19 cm in diameter (the diameter being chosen in order that it resonates at the frequency of the transmitter), the plane of the circular antenna being held substantially tangential to the curvature of the patient's body, and within about 10 cm from the patient's skin. Such a circular antenna may emit predominantly H-wave microwave radiation of wavelength between about 65 and 75 cm (most preferably about 69 cm) towards the patient's body.

While the presence of E-wave radiation generated by dipole antennae (folded or of other configuration) will not necessarily hinder the efficacy of the radiation, it can lead to greater heating of the patient's skin and a poorer depth dose than a circular or loop antenna; such effects are undesirable and dipole antennae are therefore less preferred.

Each antenna is suitably connected to a UHF generator by a coaxial cable. Dipole antennae require circulators to prevent adverse interaction between antennae. Circular or loop antennae, which can be tuned accurately to resonance at the frequency of the generator, do not require circulators. Each generator may if desired be set at a slightly different frequency at or around a central frequency in the range already mentioned (preferably about 434 MHz). Each antenna is preferably energised at about 400 W.

All equipment within about 2 metres of the antennae should be of non-metallic materials such as wood or plastic, including for example the patient support table.

Administration of the microwave radiation in the *in vitro* method of the invention may be performed by any appropriate microwave emitting device, as will be readily understood by a worker of ordinary skill in this art, provided that steps are taken to prevent overheating of the material being treated.

The following Example is included purely for ease of understanding of the present invention, and not for limitation of the scope of the invention:

### EXAMPLE

### Compositions and administration volumes

Aqueous solutions of methyl cysteine sulfoximine are prepared by dissolving 50 grams of the sulfoximine in 1 litre of normal (0.9%) saline. 20-100 ml of this solution (delivering an approximate 1 to 5 gram dose of the sulfoximine) is given orally or intravenously.

Aqueous solutions of methyl, ethyl, propyl and butyl cysteine sulfoxide are prepared by dissolving 50 gram of the respective sulfoxide in 1 litre of normal (0.9%) saline. 30-60 ml of this solution (delivering an approximate 15 mg per kilogram body weight dose of the sulfoxide) is given intravenously. Most preferably, the ethyl and butyl solutions are used in conjunction with the solution of methyl cysteine sulfoximine.

### Microwave Therapy

The methyl cysteine sulfoximine solution, optionally with the sulfoxide solution, is administered by oral administration or intravenous infusion. The intravenous route should be used when a sulfoxide solution is used. Then (within three minutes) Ultra High Frequency microwave radiation is commenced and is delivered over the next 20 - 30 minutes. 1 or 2 rest intervals of 2 to 3 minutes each are permitted during this application.

This can be repeated daily as often as is necessary to cause the desired resolution of part or all of the cancer, enhancement of the patient's T-cell count and/or reduction in the concentration of pathogenic viral and/or prion particles.

### Toxicity

No symptoms suggestive of any toxicity have been noted after the intravenous administration of the agents described, at the recommended dosages. No immediate or late changes in blood pressure, renal or liver function, electrolytes or haematology have been seen from the method.

### Contra-indications

A prime contraindication is the presence of large collections of fluid in the pleural or peritoneal cavities. Static collections of fluid which contain high concentrations of salts have heat generated in them by the application of these magnetic fields. Patients with pleural or peritoneal effusions have blockage of the lymphatics draining these areas and also involvement of the pleural and peritoneal membranes. Unless these cavities are dry (less than 100 ml fluid when treated) the heating effect aggravates the irritation of the pleural and peritoneal cavities and the effusions become worse. Recommendations for treatment of such patients are as follows:
A: Peritoneal effusions. A Le Veen shunt or equivalent should be inserted prior to treatment and must be operative so that the peritoneal cavity is kept dry. This drains the ascitic fluid back into the circulation.
B: Pleural effusions. A similar shunt can be used but is much less satisfactory. Pleural paracentesis to drain the cavity completely together with introduction of some agent causing sclerosis of the pleural cavity which obliterates it is more effective. The introduction of talc, tetracycline or another sclerosing agent appears the best way of preventing failure in the treatment.

Another contraindication to this treatment is where cancer of the stomach or bowels is causing partial obstruction to the gut. Treatment can cause breakdown of some of the cancer with a perforation of the bowel above the obstruction. All patients with bowel cancer should be fully assessed by a surgeon and if there is any evidence of obstruction the primary cancer should be removed or exteriorised by colostomy or some other similar procedure. This avoids the likelihood of perforation which can be lethal.

Regarding the HIV infections, any stage of the disease even up to frank AIDS with or without secondary infections and/or cancers are treatable. It is rational, however, to suggest that treatment when the T4 cell count is very low or zero could not raise the T4 cell count. There may be no stem cells left in the marrow, spleen, etc with which to re-populate the normal T4 cells in the blood even if some of the virus is removed.

### CASE HISTORY

### SD (a male)

Data from Western Australia show that primary malignant gliomata of the brain which come within the category of a grade 3 or 4 glioblastoma multiforme are universally fatal within one year. No conventional therapy (surgery, radiotherapy, cytotoxic chemotherapy or any combination thereof) is effective.

The following case history shows how the method of the present invention has achieved almost immediate regression and disappearance of this cancer in patient SD.

SD (a male aged 60) developed symptoms of drowsiness with discomfort in the head. Within a week he had lost some of his short and long term memory, was confused, could speak poorly with malpronunciation of words and confusion of thoughts and was increasingly unsteady in his gait. A contrast-enhanced computerised topographic (CT) scan at this stage showed a mid-brain lesion near the lateral ventricle. The position of the lesion was central in the brain, and impossible to excise without causing death. Nevertheless, a biopsy was obtained, which showed a spindle shaped glial malignancy which was immunoperoxidase-staining positive, diagnosed as a grade 3 to 4 malignant glioblastoma multiforme.

More particularly, the scan showed generally extensive non-uniform low density masses with strong enhancement after intravenous (IV) contrast, at the corpus callosum with peri-ventricular extension around the wall of the frontal horns and bodies of the lateral ventricles at both sides. Analysis of a sectioned biopsy sample showed at a macroscopic level small amounts of almost mucoid material, and at microscopic level the histology was of a fragmented tumour with spindle cells around prominent blood vessels. There was almost no residual normal cerebral tissue. A number of mitoses were seen and there was evidence of necroses.

The patient was then placed on Dexamethasone (2 mg four times a day). His condition deteriorated and he was referred for therapy according to the present invention twenty days after the CT scan.

The therapy was applied immediately as follows: intravenous injection of 5 grams S-methyl-L-cysteine sulfoximine dissolved in 100 ml of normal saline solution (no cysteine sulfoxide present), immediately followed by three applications of 434 MHz H-wave polarised electromagnetic radiation to the skull, each application lasting six minutes with two minute rest periods between each application. Four antennae were used, each energised between 433 MHz and 434.5 MHz at 400 W each.

The therapy was repeated daily for five consecutive days, followed by two rest days, and then continued again daily for three more consecutive days (eight treatments in total). The day after the final treatment the patient was reanalysed by CT scan.

The lesion affecting the corpus callosum had to a large extent been resolved. Although there was still some widening of the intraventricular portion, there was no significant contrast enhancement and very little periventricular change can be seen. The lateral ventricals remained dilated and the third and fourth ventricles were also prominent. No periventricular oedema could be seen. It was concluded that there had been a dramatic response to the therapy according to the present invention.

## Claims

1. Use of S-methyl cysteine sulfoximine of the formula 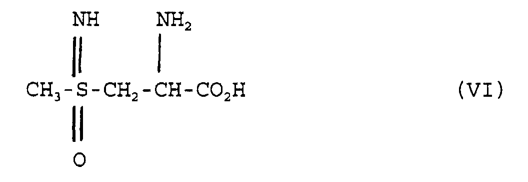 or a non-toxic salt thereof, in the preparation of a composition or compositions for use in a therapeutic method, applicable *in vivo* to a patient in which there is administered to the patient an effective amount of said sulfoximine or salt thereof, and while the said sulfoximine is present there is administered to the patient an effective dose of microwave electromagnetic radiation of frequency in the range of about 400-450 MHz.

2. A use according to claim 1, wherein the composition or compositions are for use in a method of cancer therapy in a patient.

3. A use according to claim 1, wherein the composition or compositions are for use in a method for enhancing T-cell count in an immunodeficient individual.

4. A use according to claim 1, wherein the composition or compositions are for use in a method for reducing the concentration of pathogenic viral and/or prion particles in body tissue and body fluids.

5. A use according to claim 4, for reducing the concentration of infective particulate agents selected from human immunodeficiency virus (HIV), BSE agent, scrapie agent, hepatitis agent, MS agent, CJD agent, scleroderma agent and ankylosing spondylitis agent *in vivo*.

6. A use according to any one of the preceding claims, wherein the microwave electromagnetic radiation is administered at a frequency in the range of about 432-436 MHz.

7. A modification of the use according to any one of the preceding claims, wherein, in place of or in addition to the S-methyl cysteine sulfoximine or non-toxic salt thereof, there is used a combination comprising methionine sulfoximine or a non-toxic salt thereof and methionine or a non-toxic salt thereof.

8. A use according to any one of the preceding claims, wherein the sulfoximine active agent(s) is/are used in association with one or more additional active agents selected from cystine; oxidised glutathione; non-toxic cysteine sulfoxides of formula 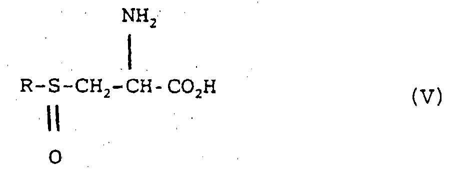 in which R is a c₁₋₄ alkyl or a C₂₋₄ alkenyl group; and non-toxic salts thereof.

9. A composition for use in a therapeutic method, applicable *in vivo* to a patient, which comprises administering to the patient an effective amount of S-methyl cysteine sulfoximine of the formula 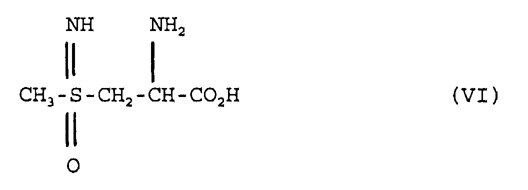 or a non-toxic salt thereof, and while said agent is present administering to the patient an effective dose of microwave electromagnetic radiation of frequency in the range of about 400-450 MHz, the composition comprising an aqueous solution of the S-methyl cysteine sulfoximine or a non-toxic salt thereof at a concentration of up to approximately 120 g/l, together with cystine and/or oxidised glutathione and/or a non-toxic sulfoxide of formula 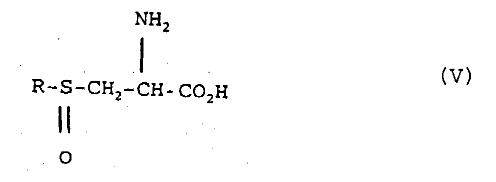 in which R is a C₁₋₄ alkyl or a C₂₋₄ alkenyl group and/or a non-toxic salt thereof.

10. A modification of the composition according to claim 9, wherein, in place of or in addition to the S-methyl cysteine sulfoximine or non-toxic salt thereof, there is used a combination comprising methionine sulfoximine or a non-toxic salt thereof and methionine or a non-toxic salt thereof, optionally with or without any or all of the cystine, oxidised glutathione, sulfoxide of formula V and/or non-toxic salt thereof.

11. A kit of two or more compositions containing active agents selected from compounds of formulae 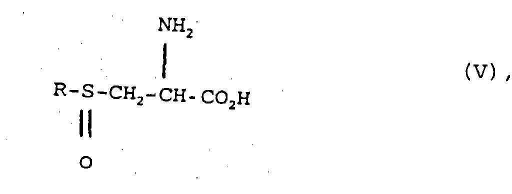 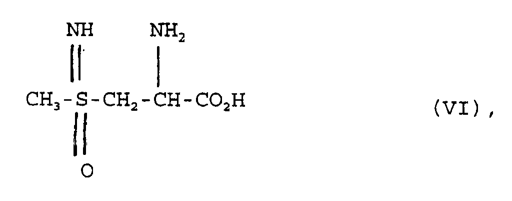 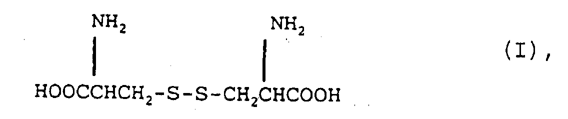 and 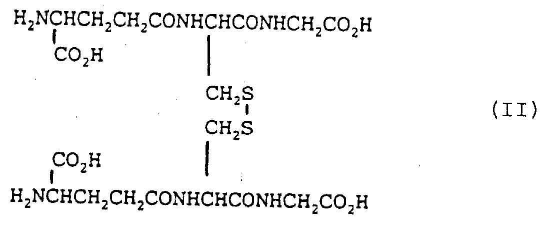 and non-toxic salts thereof, in which R is a C₁₋₄ alkyl or a C₂₋₄ alkenyl group, for rapidly sequential administration in a therapeutic method, applicable in vivo to a patient which comprises administering to the patient an effective amount of the compound of formula VI or a non-toxic salt thereof, and while said compound of formula VI is present administering to the patient an effective dose of microwave electromagnetic radiation of frequency in the range of about 400-450 MHz; a first composition of the kit comprising an aqueous solution containing a first agent selected from the agents as defined above, and a second composition of the kit comprising an aqueous solution containing a second agent selected from the agents as defined above, with the proviso that at least one of said first and second active agents is the compound of formula VI or a salt thereof; the said first and second compositions being optionally together with further composition(s) and instructional literature for the therapy.

12. A kit according to claim 11, wherein the active agents are selected from compounds of formulae V and VI and non-toxic salts thereof.

13. A modification of the kit according to claim 11 or 12, wherein, in place of or in addition to the S-methyl cysteine sulfoximine of formula VI or non-toxic salt thereof, there is used a combination comprising methionine sulfoximine or a non-toxic salt thereof and methionine or a non-toxic salt thereof, optionally with or without any or all of the compound of formula I, II, V and/or non-toxic salt thereof.
